# EUROPEAN PATENT APPLICATION

(11) **EP 1 897 535 A1**
(43) Date of publication of application: **12.03.2008**
(21) Application number: 06076644.1
(22) Date of filing: 30.08.2006
(51) Int. Cl.: A61K 9/08, A61K 33/42

(54) **Substitution fluid for haemofiltration**

(71) Applicant: Dirinco AG, 3000 Bern 25 (CH)
(72) Inventor: Kuiper, Roelof, 5404 KH Uden (NL)
(74) Representative: van Loon, C.J.J.

(57) **Abstract**

An aqueous substitution infusion fluid for haemofiltration comprising citrate. The fluid may for instance by used in a haemofiltration process comprising
- introducing blood from a subject into an extracorporeal circuit;
- adding a fluid according to any one of the claims 1-5 to the blood; thereafter
- filtering the blood using a haemofilter; and
- returning the filtered blood to the subject.

## Description

The invention relates to an aqueous substitution infusion fluid for haemofiltration and to the use of such fluid.

Acute renal failure occurs in a large number of critically ill patients (about 20%) and is associated with increased morbidity and mortality ( about 50%), despite of modern renal replacement techniques.

Continuous Haemofiltration is a continuous renal-replacement therapy (CRRT) widely used to treat patients with acute renal failure. There are several acute haemofiltration techniques and variations, but CVVH (continuous veno-venous haemofiltration) is the most commonly used one.

In CVVH the supply from and return to the blood of the patient is made via a venous access, using a blood pump to provide the driving force for the transport of blood from the patient into the filter and back to the patient.

Typically, a haemofilter, is used in CVVH. Such filter usually comprises a high flux semi-permeable membrane. The membrane may be a hollow-fibre membrane or it may be a flat membrane. It is connected to a patient's bloodstream by an extracorporeal circuit, during use. The semi-permeable membrane allows selective removal of substances (filtrate) in the blood to cross the membrane from the blood compartment into the filtrate compartment, mimicking the natural filtering function of a kidney. This leads to a considerable loss of fluid from the blood, which is removed as filtrate.

Therefore, in known haemofiltration techniques, a substitution infusion fluid is added to the blood stream in the extracorporeal circuit before it re-enters the patient's vein. Thereby, the blood volume of the patient is kept at a desired level.

In order to avoid coagulation of blood entering the extracorporeal circuit, a anti-coagulant is needed to prevent premature clot formation and haemofilter dysfunction. Heparin is commonly used for that purpose, but carries a serious risk of bleeding complications and heparin induced thrombocytopenia. Therefore, in a subgroup of critically ill patients systemic anticoagulation is absolutely contra-indicated.

It has been proposed to use citrate as an anticoagulant to reduce clogging of the filter and to prevent bleeding complications. However, it carries the risk of hypocalcaemia, when citrate is inappropriately or insufficiently counteracted by calcium infusion after passage of blood through the filter. In addition, if too much citrate enters the circulation, a metabolic alkalosis may develop, since citrate is converted to bicarbonate by the liver.

Moreover, continuous filtration techniques may attenuate a potentially harmful exaggerated immune response, particularly when high volume filtration (>6 l/hr) is used. Also, the type of anticoagulant may modulate immune responses, as known from biocompatibility studies in intermittent haemodialysis.

USA 6,743,191 describes a substitution infusion fluid for a post-dilution hemofiltration process wherein citrate is used as an anticoagulant. The citrate anticoagulant is administered separately from the substitution fluid in a concentrated solution (typically over 100 mmol/l, according to the best mode in a concentration of 500 mmol/l).

It is an object of the present invention to provide a novel substitution infusion fluid for haemofiltration which may be used as an alternative to known substitution infusion fluids, which fluid preferably overcomes one or more of the above identified problems.

In particular, it is an object of the invention to provide a substitution infusion fluid for haemofiltration which can be used in combination with relatively simple equipment, that contributes to a decreased morbidity and/or mortality of a subject treated with haemofiltration, and/or that may contribute to a prolonged adequate functioning of the haemofilter.

One or more other objects which may be solved in accordance with the invention will become apparent from the description and claims, below.

The inventors have realised that it is possible in haemofiltration to combine an anticoagulant and a substitution infusion fluid in a single composition.

Accordingly, the present invention relates to an aqueous substitution infusion fluid for haemofiltration containing 1-50 mmol/l citrate.

In addition, usually one or more inorganic electrolytes are present, in particular one or more electrolytes selected from sodium, magnesium, potassium and chloride. Normally, the fluid is essentially free of calcium ions, as calcium ions may form a complex with the citrate, thereby making it unavailable or at least less effective for acting as anticoagulant.

In addition, usually one or more carbohydrates are present, in particular glucose.

The fluid is "aqueous" , i.e. water is the major solvent, preferably the only solvent. In principle one or more other solvents may be present, provided that these are physiologically acceptable.

The term "citrate" includes fully ionised citrate (Cit³⁻) and any bound form of citrate, in particular any citrate complex, any citrate ester, any partially protonated citrate (HCit²⁻ and H₂Cit⁻) and fully protonated citrate (*i.e.* citric acid, H₃Cit). Preferably, the citrate is predominantly composed of citric acid and/or a citrate salt, in particular trisodium citrate.

In particular, the invention relates to such fluid for medical use, in particular for treating a human or another mammal. More in particular, the invention relates to the use of an aqueous infusion fluid for haemofiltration comprising 1-50 mmol/l citrate and optionally one or more other electrolytes, like ions such as sodium, magnesium, potassium, chloride, and optionally glucose in the manufacture of an aqueous substitution infusion fluid for treatment of a subject suffering from a renal dysfunction, in particular an acute renal failure or a chronic renal insufficiency.

Further, the fluid of the invention may in particular be used in the manufacture of an aqueous substitution infusion fluid for treatment of a subject suffering from a dysfunction of an organ as a result of Sepsis or Multi Organ Failure (MOF).

Further, the invention relates a filtration process for blood, comprising adding an aqueous substitution infusion fluid for haemofiltration comprising 1-50 mmol/l citrate, and optionally one or more other electrolytes, like ions such as sodium, magnesium, potassium, chloride, and optionally glucose, to blood before the haemofilter (*i.e.* Pre-dilution mode) and thereafter filtering the blood, by haemofiltration.

It has surprisingly been found that by first diluting blood with the described substitution fluid and thereafter filtering the blood (pre-dilution) it is possible to efficiently purify blood in a renal replacement technique. In addition, it is the inventors' finding that the life-time of the filter is increased in such a process and/or the risk of bleeding complications is low.

It is further advantageous that the citrate is added to the blood in a relatively high flow rate, compared to a process wherein more concentrated anticoagulant is added. High flow rates (*e.g.* of about 1-3 1. per hour) are easier to control than low flow rates, such as flow rates of a few ml. per hour.

Suitable equipment for carrying out a process (comprising the use of a fluid) of the invention is known in the art. For instance a Edwards Life Sciences Aquarius, Hospal Prisma/Prismaflex, or a Braun Diapact haemoflitration machine may be used. Also several Hemodialysis machines for chronic patient treatment are suitable, as will be understood by the skilled person.

A subject may in particular be treated with a haemofiltration process of the invention comprising
- introducing blood from the subject into an extracorporeal circuit;
- adding the aqueous substitution infusion fluid of the invention (before entering the haemofilter) (*i.e.* predilution) to the blood;
   thereafter
- filtering the blood using a haemofilter; and
- returning the filtered blood to the subject.
The filtrate is usually discharged.

The process may in particular be a continuous haemofiltration, preferably a continuous veno-venous haemofiltration (CVVH).

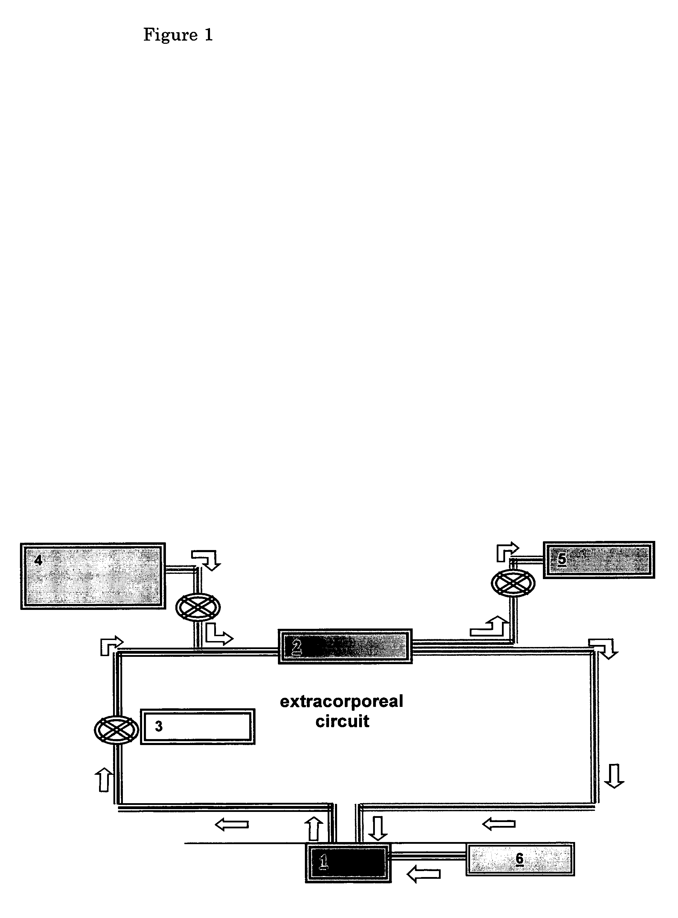

Figure 1 schematically shows a system that is particularly suitable for carrying out such a method. Herein, blood is extracted from a blood vessel of patient 1 and led to haemofilter 2 via a conduit. Suitable conduits are known in the art, for instance a double lumen catheter, such as an 11F double lumen catheter (Extraflow, Joline), may be used. The conduit is preferably inserted via a central vein, such as a jugular, subclavian or femoral vein. A blood pump 3 is normally used to pump the blood to the filter. Prior to entering the filter the substitution fluid is added to the blood from reservoir 4.

The rate of the substitution fluid flow is preferably coupled to the blood flow. Usually, the fluid is added in a volume to volume ratio (fluid to blood) in the range of 1 : 4 to 1 : 5.

The substitution fluid is preferably heated to about 37 °C or slightly higher, in particular to a temperature of 37-39 °C, in order to maintain normothermia in the subject.

In the haemofilter 2, undesired substances in the blood are selectively removed as the filtrate and discharged (5), mimicking the functioning of a kidney. The filtered blood is returned to a blood vessel of the patient 1, preferably to a vein, more preferably via a central vein, such as a jugular, subclavian or femoral vein. In case a double lumen catheter is used, the extraction and return may be accomplished via a single catheter. In particular in a haemofiltration process making use of a fluid according to the invention, wherein blood is returned to the subject, calcium ions should be added to the blood after filtering or administered directly intravenously to the subject in a sufficient amount to form a complex with excess citrate, in order to reduce the risk of hypocalcaemia reduced or even completely avoid the risk of hypocalcaemia.

Calcium ions (for instance in the form of calcium glubionate) may be administered to the patient via a separate intravenous dosage unit 6, such as a calcium drip. The skilled person will be able to determine a suitable rate for the administration of calcium based on common general knowledge and the information disclosed herein. As a rule of thumb, in particular in case calcium glubionate (0,225 mmol/ml) is used, the volume ratio of the substitution solution to the calcium globionate is usually between 150 : 1 and 200 : 1 (based on a substitution fluid comprising about 13.3 mmol/l citrate). Suitable ratio's for other calcium and/or citrate concentration can be routinely calculated, from this.

In the course of the filtration process, post filter ionised calcium (systemic ionised calcium) may be measured once (*e.g*. after 1hr) or more often (intermittently or continuously) to assess the adequacy of anticoagulation. If desired, the flow rate of the substitution fluid and/or the calcium drip may be adjusted. See also the Example.

For use in haemofiltration, the aqueous substitution infusion fluid should generally be safe to intravenously administer to a human, another mammal or another vertebrate, in particular a patient suffering from a renal dysfunction. Accordingly, it should be sterile and pyrogen-free such that it can be administered safely. As the substitution solution is given intra venously, it generally requires the quality mentioned in the European Pharmacopoeia standards.

Further, for use in haemofiltration, the pH of the infusion fluid should generally be sufficiently close to the pH of blood to avoid a too big change in pH after adding the fluid to the blood. Preferably, the pH of the substitution solution is at least 4.5. Preferably, the pH of the substitution fluid is up to 6.5 (pH is as measured in the fluid, using a pH electrode equilibrated at pH 4 and 7).

Further, for use in haemofiltration, the osmolarity of the fluid should be sufficiently low, to maintain a sufficiently low osmolarity of the blood, also after administration of the fluid. Preferably, the osmolarity is essentially isotonic or hypotonic. Accordingly, the osmolarity is preferably about 300 mOsm/l or less. To maintain a substantially normal osmolarity of the blood, the osmolarity is usually at least 200 mOsm/l.

The use of a relatively low concentration of citrate, preferably of about 40 mmol/l or less, in particular of 25 mmmol/l or less, more in particular of 20 mmol/l or less is considered advantageous in view of safety and/or prolonging the life-time of the filter.

A relatively low concentration of anticoagulant is advantageous from a safety point of view, as there is a reduced risk of overdosing of citrate. Usually, overdosing of citrate is avoided by adding calcium after filtering the blood, but in practice often equipment is used wherein the dosage system for anticoagulant (pre-filter) and the dosage system for calcium (post-filter) are not controlled in combination. Thus, as the present invention also allows use of citrate in a relatively low concentration (for values, see above), the risk of overdosing citrate upon malfunction of the calcium dosage system, is reduced, in comparison to a system wherein high concentrations of citrate are used.

For practical reasons, the citrate concentration preferably is at least 5 mmol/l, in particular at least 10 mmol/l.

Very good results have been achieved with a substitution fluid having a concentration of about 13 mmol/l/

In addition to citrate, the fluid contains counter ions, in particular sodium ions. Further, preferably potassium ions and/or magnesium ions are present. Chloride is usually present to keep electrochemical balance.

Sodium ions are usually present in a concentration of 50 mmol/l or more. Sodium ions are preferably present in a concentration of at least 100 mmol/l, more preferably in a concentration of at least 125 mmol/l. A concentration of at least 139 mmol/l is in particular preferred.

For a desirably low osmolarity, the sodium ion concentration is usually 150 mmol/l or less, preferably 145 mmol/l or less.

If present, the concentration of potassium ion is usually at least 0.5 mmol/l, preferably at least 1.0 mmol/l. Usually the concentration is 5 mmol/l or less, preferably 4 mmol/l or less. If present, the concentration of magnesium ion is usually at least 0.25 mmol/l, preferably at least 0.4 mmol/l. Usually, the concentration is 1.25 mmol/l or less, preferably 1.0 mmol/l or less.

The fluid is preferably essentially free of calcium ions, as calcium ions form a complex with citrate, thereby inactivating the anti coagulating function of citrate. In practice, trace amount of calcium ions may be present (e.g. as a result of their presence as an impurity in the raw materials used to prepare the fluid) In particular, the calcium ion concentration usually is less than 1.0 mmol/l, more in particular less than 0.5 mmol/l, preferably less than 0.1 mmol/l.

For providing an energy source to the subject, when the filtered blood is returned, a monosaccharide may be present in the fluid, preferably glucose. If present, the concentration is usually in the range of 1-15 mmol/l, preferably in the range of 3-10 mmol/l.

The invention will now be illustrated by the following example.

### Example

The replacement solution is delivered pre filter (predilution mode). Patients on regional citrate anticoagulation have a separate intravenous calcium drip 6 (Figure 1). In this Example, calcium glubionate (0,225 mmol/ml), because this solution can be administered through a peripheral line. The rate of citrate infusion (and thus predilution fluid) is coupled to the blood flow. Post filter ionised calcium (systemic ionised calcium) is measured continuously, intermittently or initially and after a specific period of time (*e.g.* after 1 hour) to assess the adequacy of anticoagulation. The citrate administration with this method is enough to produce an ionised calcium in the extracorporeal circuit lower than 0,3 mmol/l. The systemic calcium administration depends on the systemic ionised calcium as outlined in Tables 1 to 3.

**Table 1. Initial pump settings/Ionised calcium 1.0 - 1.1 mmol/l.**

| Blood pump (ml/min) | Citrate-substitution flow (ml/hr) | Calcium pump (ml/hr) |
|---|---|---|
| 140 | 1900 | 9,5 |
| 160 | 2100 | 10,5 |
| 180 | 2400 | 12 |
| 200 | 2700 | 13,5 |

Initially, every subject is treated with a process making use of settings indicated in Table 1.

After 1 hour of treatment, the systemic ionised calcium concentration is determined.

In case the concentration is 1.0-1.1 mmol/l, the treatment is continued using settings as given in Table 1.

If the systemic ionised calcium concentration is 0.9-1.0, the settings are changed to settings as given in Table 2.

If the systemic ionised calcium concentration is 0.8 - 0.9 mmol/l, the settings are changed to settings as given in Table 3.

In case the concentration is 1.1 - 1.2 mmol/l, the settings are changed to settings as given in Table 4.

If the systemic ionised calcium concentration is outside the 0.8 - 1.2 mmol/l range, the attending nephrologist should be consulted.

If the ionised calcium is getting worse (by more than 0.1 mmol/l), it should be checked whether citrate accumulation occurs and the ionised calcium levels should be determined every 3 hours.

**Table 2: Low ionised calcium 0.9 - 1.0 mmol/l**

| Blood pump (ml/min) | Citrate-substitution flow (ml/hr) | Calcium pump (ml/hr) |
|---|---|---|
| 140 | 1900 | 11 |
| 160 | 2100 | 12,5 |
| 180 | 2400 | 14 |
| 200 | 2700 | 16 |

**Table 3: Low ionised calcium 0.8 - 0.9 mmol/l.**

| Blood pump (ml/min) | Citrate-substitution flow (ml/hr) | Calcium pump (ml/hr) |
|---|---|---|
| 140 | 1900 | 15 |
| 160 | 2100 | 16.5 |
| 180 | 2400 | 18 |
| 200 | 2700 | 20 |

**Table 4: High ionised calcium 1.1- 1.2 mmol/l.**

| Blood pump (ml/min) | Citrate-substitution flow (ml/hr) | Calcium pump (ml/hr) |
|---|---|---|
| 140 | 1900 | 7.5 |
| 160 | 2100 | 8.5 |
| 180 | 2400 | 10 |
| 200 | 2700 | 11 |

## Claims

1. An aqueous substitution infusion fluid for haemofiltration comprising 1 to 50 mmol/l citrate and one or more electrolytes, in particular one or more electrolytes selected from sodium, potassium, magnesium and chloride.

2. A fluid according to claim 1, which is essentially free of calcium ions.

3. A fluid according to any of the preceding claims, comprising
1-50 mmol/l citrate;
50-150 mmol/l sodium ions;
0.5-5 mmol/l potassium ions;
0.25-1.25 mmol/l magnesium ions;
1-15 mmol/l glucose; and
chloride to keep electrochemical balance

4. A fluid according to any one of the preceding claims, wherein the citrate concentration is 5-40 mmol/l, preferably 10-25 mmol/l, more preferably about 13 mmol/l.

5. A fluid according to any of the preceding claims for medical use.

6. Use of a fluid according to any of the claims 1-5 in the manufacture of an aqueous substitution infusion fluid for treatment of a subject suffering from a renal dysfunction, in particular an acute renal failure or a chronic renal sufficiency.

7. Use of a fluid according to any of the claims 1-5 in the manufacture of an aqueous substitution infusion fluid for treatment of a subject suffering from a dysfunction of an organ as a result of Sepsis or Multi Organ Failure.

8. Use according to claim 6 or 7, wherein the aqueous substitution infusion fluid is for a treatment comprising diluting blood taken from the subject, with the fluid, prior to filtering the blood.

9. Use according to any of the claims 6-8, wherein the medicament or medical aid is for treatment of the subject by continuous veno-venous haemofiltration.

10. Use of a fluid according to any of the claims 1-5 in the manufacture of a medicament or medical aid for reducing or avoiding the risk of bleeding complications in a subject which is subjected to haemofiltration.

11. Use of a fluid according to any of the claims 1-5 for avoiding coagulation of blood in a haemofilter.

12. Use of a fluid according to any of the claims 1-5 for prolonging the life-time of a haemofilter.

13. Haemofiltration process, comprising *in vitro* adding a fluid according to any of the claims 1-5 to blood and thereafter filtering the blood.

14. Haemofiltration process comprising
- introducing blood from a subject into an extracorporeal circuit;
- adding a fluid according to any one of the claims 1-5 to the blood; thereafter
- filtering the blood using a haemofilter; and
- returning the filtered blood to the subject.

15. Haemofiltration process according to claim 14, wherein calcium ions are added to the filtered blood prior to returning the blood or wherein calcium ions are intravenously administered separately from the filtered blood.
